## Europäisches Patentamt

## European Patent Office

## Office européen des brevets

(19)

(11) Publication number: **0 168 358**
**B1**

# EUROPEAN PATENT SPECIFICATION

(12)

(45) Date of publication of the patent specification:
**10.08.88**

(51) Int. Cl.⁴: **C 07 C 39/04,** C 07 C 37/52

(21) Application number: **85830144.3**

(22) Date of filling: **12.06.85**

(54) **Method for the pyrolysis of phenolic pitch.**

(30) Priority: **15.06.84 IT 2142684**

(43) Date of publication of application:
**15.01.86 Bulletin 86/3**

(45) Publication of the grant of the patent:
**10.08.88 Bulletin 88/32**

(84) Designated Contracting States:
**AT BE CH DE FR GB LI LU NL SE**

(56) References cited:
**DE-B-1 493 508**
**US-A-3 091 646**

(73) Proprietor: **ANIC S.p.A., Via Ruggero Settimo, 55,
I-90139 Palermo (IT)**

(72) Inventor: **Messina, Giuseppe, Via Perpignan, 27,
I-07041 Alghero (Sassari) (IT)**
Inventor: **Moretti, Mario Domenico, Via Oriani, 5,
I-07100 Sassari (IT)**
Inventor: **Sanna, Salvatore Roberto, Via Biasi, 1,
I-07037 Sorso (Sassari) (IT)**

(74) Representative: **Perani, Aurelio, c/o JACOBACCI-
CASETTA & PERANI S.p.A 7, Via Visconti di
Modrone, I-20122 Milano (IT)**

## Description

The present invention relates to a method for the pyrolysis of phenolic pitch coming from plants for the production of phenol from cumene, via cumene hydroperoxide. It is known that, in the method for the production of phenol from cumene _via_ cumene hydroperoxide, the cumene is oxidised to cumene hydroperoxide and the latter is subjected to acid cleavage to obtain a liquid reaction mixture containing phenol and acetone as the main constituents. This mixture, generally termed the acid cleavage product of cumene hydroperoxide, is neutralised and fractionally distilled to separate a head fraction containing the acetone and a bottom fraction containing the phenol.

The acetone fraction is further fractionated to separate the acetone from a hydrocarbon fraction constituted mainly by cumene and alpha-methylstyrene.

The acetone is purified to obtain this compound with the desired degree of purity. The hydrocarbon fraction may be treated to separate the cumene (for recycling to the oxidation stage) from the alpha-methylstyrene. Alternatively the hydrocarbon fraction is subjected to catalytic hydrogenation to convert the alpha-methylstyrene into cumene and the product may be recycled to the cumene oxidation stage after purification.

The fraction containing the phenol is, in its turn, fractionally distilled and subjected to a series of physical and chemical treatments so as finally to separate the purified phenol as the head product from a bottom product generally known as phenolic pitch. This phenolic pitch is a complex mixture containing phenol, acetophenone, cumylphenols, dimethylphenyl carbinol, dimers of alpha-methylstyrene and heavier products formed during the oxidation of cumene and/or in the acid cleavage of the cumene hydroperoxide and/or during the treatments for separation of the acid cleavage products of cumene hydroperoxide.

It is known in the art to pyrolyse phenolic pitch at high temperature in order to separate a light fraction containing cumene, alpha-methylstyrene, phenol and acetophenone, from a heavy fraction of a tarry consistency. The light fraction thus obtained may in its turn be distilled to separate hydrocarbons together with part of the phenol content at the head and the minimum boiling point azeotropic mixture acetophenone-phenol at the bottom. The phenol is then separated from these fractions for example by extraction with aqueous sodium hydroxide solutions.

The pyrolysis of the phenolic pitch may be carried out thermally or catalytically as described, for example, in U.S. Patents Nos. 2.628.983, 2.671.809, 2.715.145, 2.728.795, 2.736.753, 3.110.744, 3.187.052 and 3.365.375.

In particular, the thermal pyrolysis requires temperatures in the range 300 to 400° C.

However, at the higher values within this range, the light products from the pyrolysis are rich in various impurities which are difficult to separate and in any case undesirable, resulting from collateral fragmentation reactions.

It is also found that coke forms in the reactor used for the pyrolysis. On the other hand, at the lower temperatures within the range indicated above, the rate of pyrolysis is undesirably low (U.S. Patent No. 3.850.996).

In order to avoid these disadvantages recourse is made in the art to acidic catalysts which are generally soluble in the phenolic pitch and allow the pyrolysis to be carried out at acceptable rates at relatively low temperatures so as to improve the purity of the light products and reduce the formation of coke in the reactor.

these acid catalysts have not shown themselves to be completely satisfactory in that they give rise to ̣ion and the presence of free acid in the light distillate which is responsible for condensation reaction between the light products.

It has thus been proposed to use other catalysts which are favouring the pyrolysis of the phenolic pitch without or substantially without the disadvantages typical of acid catalysts. Thus, for example, in German Patent No. 1.493.508, aluminium phenate is used as the pyrolysis catalyst for phenolic pitch, it being obtainable, for example, by the dissolution of aluminium metal in phenol. This catalyst however displays its activity at a relatively high concentration range such that the quantity of aluminium present in the pyrolysis residue is of the order of 1500-2000 parts per million. This means that the pyrolysis residue cannot be disposed of by combustion because of the formation of metal slag in the combustion chamber.

It would therefore, be desirable to provide a pyrolysis catalyst which does not have the disadvantages of acidic catalysts but which is active in the pyrolysis of phenolic pitch in concentrations sufficiently low to eliminate or at least substantially reduce, the disadvantages of aluminium phenate. The present invention is based essentially on the finding that by bringing phenolic pitch into contact with solid metallic aluminium, under particular conditions of temperature and time, one can cause a quantity of aluminium of the order of 50-100 parts per million to dissolve in the pitch and this dissolved aluminium constitutes an extremely active catalyst in the pyrolysis of the phenolic pitch at relatively low temperatures.

Accordingly, in the present invention, phenolic pitch resulting as the by product of processes for the production of phenol from cumene via cumene hydroperoxide, containing phenol, acetophenone, cumylphenols, dimethylphenyl carbinol and dimers of alpha-methylstyrene, as well as heavier products and with a phenol content of less than about 10 % by weight, is brought into contact with solid metallic aluminium at temperatures of from 150 to 250° C, for periods of from 2 to 4 hours, so as to cause a quantity of aluminium of 50 to 100 parts per million to dissolve in the pitch itself and the pitch thus obtained is then heated to a temperature of from 250 to 350° C for a period of from 2 to 10 hours to pyrolyse the pitch and obtain a light fraction constituted essentially by acetophenone, phenol, cumene and alpha-methylstyrene and a heavy fraction of tarry material.

Phenolic pitch with a phenol content greater than about 10 % by weight is unsuitable for the pyrolysis process of the present invention in that it dissolves an undesirably high quantity of aluminium, in any case greater than the range of values indicated above. Hence such phenolic pitch is conveniently pretreated by distillation or evaporation so as to bring its phenol content to a value below about 10 % by weight, in particular between about 5 and about 10 % by weight.

According to one embodiment of the present invention, the phenolic pitch is made to flow over a fixed bed of solid aluminium particles, operated with a contact time of from 2 to 4 hours and a temperature of from 150° to 250°C, so as to obtain a phenolic pitch containing about 50-100 parts per million of dissolved aluminium, suitable for subsequent pyrolysis. According to another embodiment of the present invention, the phenolic pitch and the powdered aluminium metal are kept in contact, preferably under agitation, under the conditions of time and temperature indicated above to obtain a phenolic pitch suitable for the pyrolysis treatment.

According to the method of the present invention, the pyrolysis of the phenolic pitch containing about 50 to 100 parts per million of dissolved aluminium is carried out by heating the pitch itself to a temperature of 250 to 350°C for a period of 2 to 10 hours.

In the preferred embodiment, this is carried out at a temperature of from 280 to 320°C for a period of from 2 to 5 hours.

Under these conditions, a light fraction is produced which is constituted essentially by acetophenone, phenol, cumene and alpha-methylstyrene, with only small quantities of dimers of alpha-methylstyrene and cumylphenols.

The pyrolysis is conveniently carried out in a boiler surmounted by a filled column, the latter serving essentially to avoid sprays or entrainment of heavy compounds.

The pyrolysis yields of cumylphenols and dimers of decomposed alpha-methyl styrene, evaluated as molar percents are a function of the temperature and of the residence time under the pyrolysis conditions and, for the conditions indicated above, generally vary from about 70 to 95 %.

The light products formed may be distilled, preferably under pressure, to separate the acetophenone as the bottom product, this conveniently being reunited with the tarry residues from the pyrolysis to increase their fluidity. The product at the head of the distillation column may be recycled to the process for the production of phenol (acid cleavage stage or fractionation section) or may be subjected to treatments to separate and purify the various constituents.

The tarry residue from the pyrolysis, having a more or less viscose consistency depending on the severity of the pyrolysis itself, contains a quantity of residual aluminium of the order of 100-200 ppm.

One is thus dealing with a product which can easily be disposed of compared with the process which uses aluminium phenate which discharges a pyrolysis residue with an aluminium content 5 to 10 times greater.

The method of the present invention has a further advantage over the catalytic method of the prior art in that it avoids the need for a separate section for the preparation and metering of the catalyst and also uses metal aluminium which is widely available, of low cost and easy to handle.

Finally, given the catalytic activity of the aluminium, even in such small quantities, it is possible to carry out the pyrolysis of the pitch within a temperature range, and with residence times, such as to simplify the treatment itself as regards problems of heat transfer, plant operation and the formation of coke.

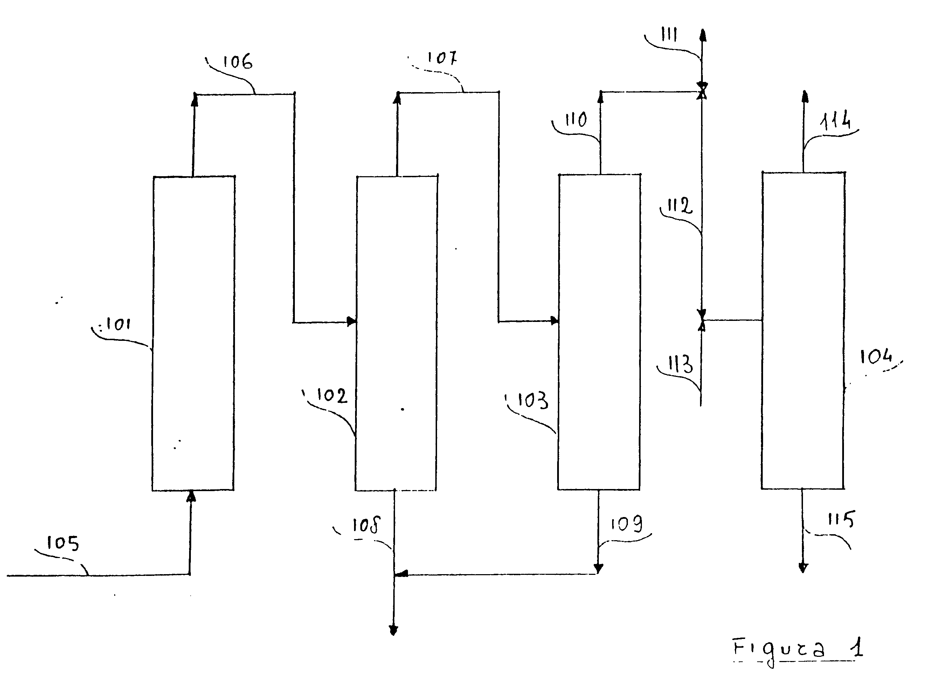

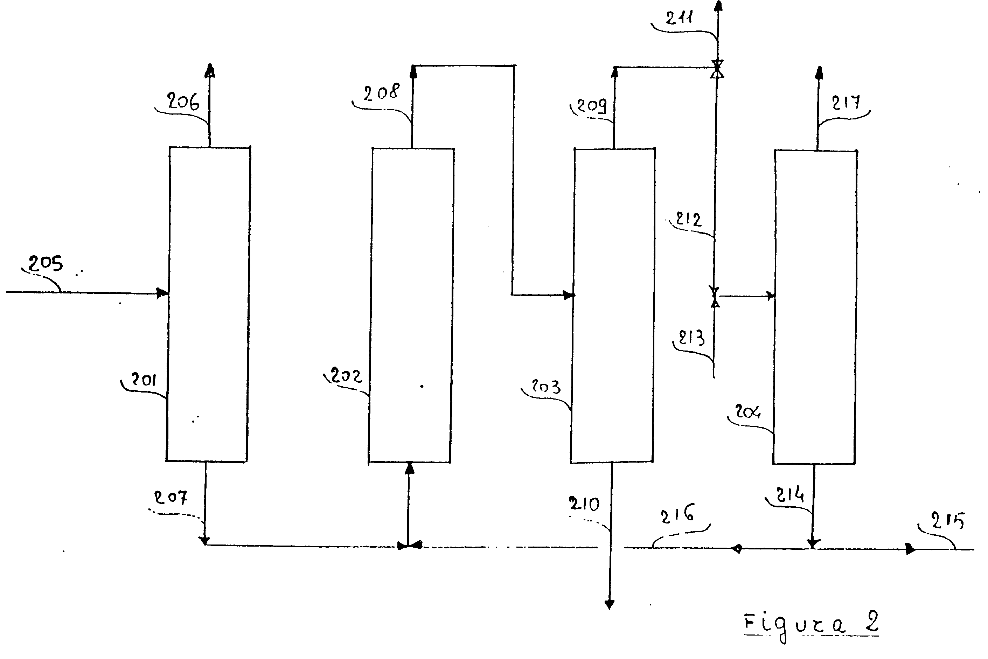

With reference to the appended drawings, Figures 1 and 2 represent two possible practical embodiments of the method of the invention.

More particularly, with reference to Figure 1, phenolic pitch with a phenol content of less than about 10 % by weight, is supplied through the line 105 to the aluminium-dissolution vessel 101 which contains granular aluminium metal in the form of a fixed bed. The pitch thus treated, with an aluminium content of 50-100 parts per million, is taken from the vessel 101 through the line 106 and is fed to the pyrolysis boiler 102 from which the light pyrolysis products are discharged through the line 107 and the pyrolysis residue through the line 108. The light products from the pyrolysis are fed to the distillation column 103 which operates under pressure, with separation of acetophenone at the foot, line 109, this being reunited with the residue from the pyrolysis, and with separation of phenol and hydrocarbons at the head, line 110.

The products separated at the head of the column 103 may be conveyed to the distillation section of the phenol plant through the line 111 or may be directed through the line 112 to the column 104 together with water vapour supplied through the line 113.

In column 104 distillation is carried out with a flow of steam and the hydrocarbon components are separated at the head, line 114, and crude phenol at the foot, line 115.

With reference to Figure 2, phenolic pitch with a phenol content greater than about 10 % by weight is conveyed through the line 205 to the thin film evaporator 201. At the head of the evaporator 201, a light fraction is recovered through the line 206 which contains some of the phenol and acetophenone present in the phenolic pitch. At the foot of the evaporator 201, phenolic pitch is recovered through the line 207 with a phenol content of from about 5 to 10 % by weight, this being directed to the metal aluminium dissolution vessel 202 containing granular aluminium metal disposed in the form of a fixed bed. The pitch thus treated, with an aluminium content of 50-100 parts per million, is conveyed through the line 208 to the pyrolysis boiler 203 from which the light pyrolysis products are discharged through the line 209 and the residue from the pyrolysis through the line 210.

The light products from the pyrolysis may be conveyed to the distillation section of the phenol plant through the line 211 or may be directed to the column 204 through the line 212 together with water vapour supplied

3

through the line 213. In the column 204 distillation is carried out with a flow of steam with separation of crude phenol at the foot, line 214, this being recovered through the line 215 and, if necessary, partially recycled to the dissolution vessel 202 through the line 216. At the head of the column 204, the hydrocarbon products are recovered through the line 217.

The experimental examples which follow are illustrative of the invention.

**Example 1.**

Phenolic pitch containing:

| | |
|---|---|
| acetophenone | 11.38 % by weight |
| dimethylphenyl carbinol | 1.53 % by weight |
| phenol | 5.49 % by weight |
| dimers of alpha-methylstyrene | 8.99 % by weight |
| cumylphenols | 18.51 % by weight |
| heavy fraction | 54.10 % by weight |

is percolated through a bed of metal aluminium particles operated at 200°C with a stay time of 3.5 hours and phenolic pitch is recovered with a dissolved aluminium content of about 100 parts per million.

The phenolic pitch thus treated is pyrolysed in a pyrolysis boiler at 320°C for a period of 3 hours. In this period of time, 48.7 % by weight of the phenolic pitch is converted into light pyrolysis products, having the following composition:

| | |
|---|---|
| light fraction | 9.32 % by weight |
| water | 2.38 % by weight |
| cumene | 14.26 % by weight |
| alpha-methylstyrene | 18.89 % by weight |
| acetophenone | 24.44 % by weight |
| dimethylphenyl carbinol | 1.07 % by weight |
| phenol | 29.64 % by weight |

The pyrolysis residue has the following composition.

| | |
|---|---|
| dimers of alpha-methylstyrene | 0.90 % by weight |
| cumylphenols | 7.40 % by weight |
| heavy fraction | 92.6 % by weight |

The conversion of cumylphenols is 79.5 % molar and the conversion of the dimers of alpha-methylstyrene is 94.9 % molar.

The pyrolysis residue has an aluminium content of about 200 parts per million.

**Example 2.** (comparison)

Aluminium phenate was added to the phenolic pitch described in Example 1 in quantities such as to provide 0.09 % by weight of aluminium metal and pyrolysis was then carried out under the conditions of Example 1, conversion of 51.5 % by weight of the phenolic pitch into light pyrolysis products being obtained, these products having the following composition:

| | |
|---|---|
| light fraction | 9.42 % by weight |
| water | 2.88 % by weight |
| cumene | 12.69 % by weight |
| alpha-methylstyrene | 19.95 % by weight |
| acetophenone | 24.92 % by weight |
| dimethylphenyl carbinol | 0.16 % by weight |
| phenol | 28.99 % by weight |
| dimers of alpha-methylstyrene | 1.09 % by weight |

The pyrolysis residue had the following composition:

| | |
|---|---|
| cumene | 0.39 % by weight |
| phenol | 0.23 % by weight |
| dimers of alpha-methylstyrene | 2.08 % by weight |
| cumylphenols | 10.89 % by weight |
| heavy fraction | 86.41 % by weight |

Hence the conversion of the cumylphenols was 69.9 % molar and the conversion of the dimers of alpha-methylstyrene was 81.3 % molar.

The pyrolysis residue had an aluminium content of about 1800 parts per million.

**Example 3.** (comparison)

This was carried out as in Example 2 with the addition of 0.045 % by weight of aluminium in the form of aluminium phenate to the phenolic pitch, giving a 44.4 % by weight conversion of the phenolic pitch into light pyrolysis products having the following composition:

| | |
|---|---|
| light fraction | 9.29 % by weight |
| water | 1.56 % by weight |
| cumene | 11.29 %% by weight |
| alpha-methylstyrene | 19.20 % by weight |
| acetophenone | 28.74 % by weight |
| dimethylphenyl carbinol | 0.95 % by weight |
| phenol | 29.34 % by weight |
| dimers of alpha-methylstyrene | 0.63 % by weight |

The pyrolysis residue had the following composition:

| | |
|---|---|
| cumene | 0.44 % by weight |
| phenol | 0.19 % by weight |
| dimers of alpha-methylstyrene | 2.34 % by weight |
| cumylphenols | 16.58 % by weight |
| heavy fraction | 80.45 % by weight |

Hence the conversion of the cumylphenols was 50.5 % molar and the conversion to 78.4 % molar.

**Example 4.**

Phenolic pitch was used which was free from lower boiling constituents and had the following composition:

| | |
|---|---|
| phenol | 4.3 % by weight |
| dimers of alpha-methylstyrene | 10.96 % by weight |
| cumylphenols | 26.13 % by weight |
| heavy fraction | 58.61 % by weight |

The phenolic pitch, preheated to 80°C was supplied continuously to a cylindrical jacketed reactor (height 30 cm; diameter 50.8 mm containing granular aluminium metal. The reactor was heated with oil thermostatically controlled to 200 to 220°C and the residence time was about 3.5 hours.

The treated pitch containing about 100 parts per million of dissolved aluminium passed continuously by overflow into a pyrolysis boiler which was operated at 320°C, with a residence time of about 4 hours. The residence time in the boiler was regulated by means of a valve for discharging the pyrolysis residue located at the bottom of the boiler itself.

Under these conditions, an average conversion of 31.4 % of the phenolic pitch into light pyrolysis products was achieved, the products having the following average composition:

| | |
|---|---|
| light fraction | 10.89 % by weight |
| water | 4.28 % by weight |
| cumene | 21.22 % by weight |
| alpha-methylstyrene | 26.13 % by weight |
| acetophenone | 0.79 % by weight |
| dimethylphenyl carbinol | 0.03 % by weight |
| phenol | 36.56 % by weight |
| dimers of alpha-methylstyrene | 0.10 % by weight |

The pyrolysis residue had the following composition:

| | |
|---|---|
| phenol | 0.23 % by weight |
| dimers of alpha-methylstyrene | 2.22 % by weight |
| cumylphenols | 12.50 % by weight |
| heavy fraction | 85.05 % by weight |

Hence the conversion of the cumylphenols was 67.2 % molar and the conversion of the dimers of alpha-methylstyrene was 94.3 % molar.

The residue from the pyrolysis had an aluminium content of about 180 parts per million.

## Claims

1. Method for the pyrolysis of phenolic pitch characterised in that:
   a) phenolic pitch resulting as a by product of the processes for the production of phenol from cumene via cumene hydroperoxide, containing phenol, acetophenone, cumylphenols, dimethylphenyl carbinol, dimers of alphamethyl styrene and higher boiling products, and with a phenol content of less than about 10 % by weight, is placed in contact with solid metallic aluminium at a temperature of from 150 to 250°C for periods of from 2 to 4 hours, so as to cause a quantity of aluminium of 50 to 100 parts per million to dissolve in the pitch itself;
   b) the phenolic pitch thus treated is heated to a temperature of from 250 to 350°C for a period of from 2 to 10 hours to pyrolyse the pitch and obtain a light fraction constituted essentially by acetophenone, phenol, cumene and alpha-methylstyrene and a heavy fraction of tarry material; and
   c) the light fraction is recovered and the heavy fraction discharged with an aluminium content of about 100 to 200 parts per million.

2. Method according to Claim 1, characterised in that stage a) is carried out at a temperature of from 180 to 220°C.

3. Method according to Claim 1, characterised in that, in stage a), the phenolic pitch is made to flow over a bed of solid metal aluminium particles.

4. Method according to Claim 1, characterised in that stage b) is carried out at a temperature of from 280 to 320°C for a period of from 2 to 5 hours.

## Patentansprüche

1. Verfahren zur Pyrolyse von phenolischem Teer, dadurch gekennzeichnet, daß:

   a) phenolischer Teer, der als Nebenprodukt des Herstellungsverfahrens von Phenol aus Cumol über Cumolhydroperoxid entsteht, der Phenol, Acetophenon, Cumylphenole, Dimethylphenylcarbinol, Dimere von Alpha-Methyl-Styren und höhersiedende Produkte enthält und einen Phenolgehalt von weniger als ungefähr 10 Gewichtsprozent besitzt, mit festem metallischem Aluminium bei Temperaturen von 150°C bis 250°C über eine Dauer von zwei bis vier Stunden in Kontakt gebracht wird, so daß sich eine Menge Aluminium von 50 bis 100 ppm (Teile pro Million Teile) im Teer löst;

b) phenolischer Teer, der so behandelt wurde, auf eine Temperatur von 250°C bis 350°C für eine Dauer von zwei bis zehn Stunden erhitzt wird, um den Teer zu verbrennen und eine leichte Fraktion zu erhalten, die im wesentlichen aus Acetophenon, Phenol, Cumol und Alpha-Methyl-Styren und einer schweren Fraktion aus teerigem Material besteht; und

c) die leichte Fraktion rückgewonnen und die schwere Fraktion mit einem Aluminiumgehalt von 100 bis 200 ppm ausgetragen wird.

2. Verfahren nach Anspruch 1, dadurch gekennzeichnet, daß der Verfahrensschritt a) bei einer Temperatur von 180°C bis 220° C ausgeführt wird.

3. Verfahren nach Anspruch 1, dadurch gekennzeichnet, daß beim Verfahrensschritt a) der phenolische Teer über ein Bett von festen metallischen Aluminiumteilchen geführt wird.

4. Verfahren nach Anspruch 1, dadurch gekennzeichnet, daß der Verfahrensschritt b) bei einer Temperatur von 280°C bis 320°C für eine Dauer von zwei bis fünf Stunden durchgeführt wird.

**Revendications**

1. Procédé pour la pyrolyse d'une poix phénolique, caractérisé en ce que:

a) on met en contact une poix phénolique formée comme sous-produit des procédés pour la production de phénol à partir de cumène par l'intermédiaire de l'hydroperoxyde de cumène, contenant du phénol, de l'acétophénone, des cumylphénols, du diméthylphénylcarbinol, des dimères d'α-méthylstyrène et des produits de point d'ébullition plus élevé, et ayant une teneur en phénol de moins d'environ 10 % en poids, avec de l'aluminium métallique solide à une température de 150 à 250°C pendant des durées de 2 à 4 h, de manière à provoquer la dissolution d'une quantité d'aluminium de 50 à 100 parties par million dans la poix proprement dite;

b) on chauffe la poix phénolique ainsi traitée à une température de 250 à 350°C pendant une durée de 2 à 10 h pour pyrolyser la poix et obtenir une fraction légère constituée essentiellement d'acétophénone, de phénol de cumène et d'α-méthylstyrène et une fraction lourde de produit goudronneux; et

c) on récupère la fraction légère et on décharge la fraction lourde ayant une teneur en aluminium d'environ 100 à 200 parties par million.

2. Procédé selon la revendication 1, caractérisé en ce que l'étape a) est mise en oeuvre à une température de 200 à 220°C.

3. Procédé selon la revendication 1, caractérisé en ce que dans l'étape a) on fait couler la poix phénolique sur un lit de particules solides d'aluminium métallique.

4. Procédé selon la revendication 1, caractérisé en ce que l'étape b) est mise en oeuvre à une température de 280 à 320°C pendant une durée de 2 à 5 h.

Figura 1

Figura 2

0 168 358